(19) Europäisches Patentamt
European Patent Office
Office européen des brevets

(11) **EP 1 545 265 B1**

(12) **EUROPEAN PATENT SPECIFICATION**

(45) Date of publication and mention
of the grant of the patent:
**28.10.2009 Bulletin 2009/44**

(51) Int Cl.:
*A46B 11/00* (2006.01)    *A61Q 11/00* (2006.01)
*A61C 17/22* (2006.01)

(21) Application number: **03808099.0**

(22) Date of filing: **30.09.2003**

(86) International application number:
**PCT/US2003/030815**

(87) International publication number:
**WO 2004/032674 (22.04.2004 Gazette 2004/17)**

(54) **ORAL COMPOSITIONS AND USE THEREOF**

ORALE ZUSAMMENSETZUNGEN UND DEREN VERWENDUNG

COMPOSITIONS ORALES ET LEUR UTILISATION

(84) Designated Contracting States:
**AT BE BG CH CY CZ DE DK EE ES FI FR GB GR
HU IE IT LI LU MC NL PT RO SE SI SK TR**

(30) Priority: **04.10.2002 US 416067 P
31.10.2002 US 422828 P
13.06.2003 US 478653 P**

(43) Date of publication of application:
**29.06.2005 Bulletin 2005/26**

(73) Proprietor: **THE PROCTER & GAMBLE COMPANY
Cincinnati, Ohio 45202 (US)**

(72) Inventors:
• **CORCORAN, Ruth, Ann
Surbiton, Surrey KT6 7LF (GB)**
• **DAY, Trevor Neil
Egham,
Surrey TW20 0JP (GB)**
• **HUNT, Sheri, Anne
West Chester, OH 45069 (US)**

(74) Representative: **Wilding, Richard Alan et al
Procter & Gamble Technical Centres Limited
Patent Department
Rusham Park
Whitehall Lane
Egham, Surrey TW20 9NW (GB)**

(56) References cited:
EP-A- 0 449 457          WO-A-00/03676
WO-A-02/11641          WO-A-02/41802
DE-A- 19 740 453          US-A- 3 538 230
US-A- 4 420 312          US-A- 5 178 869
US-A- 5 192 529

## Description

**Technical Field**

Oral compositions for use in integrated oral treatment systems are provided.

**Background**

Recently, electric toothbrushes have become very popular with consumers and dentists alike. These devices are thought to provide better cleaning and massaging of the oral surfaces than traditional manual brushing. However, electric toothbrushes still require the dispensing of dentifrice onto the bristles prior to use. Commencement of brushing quickly results in a decrease of the concentration of dentifrice on the bristles, and possibly results in lower than expected cleaning of the tooth surfaces.

As a result, it is desirable to have an oral treatment system that comprises an oral composition that is dispensed through the bristle head and onto the bristles during brushing either automatically, or at will. Such an idea is not new. For example, US 3,217,720, WO 02/41801, WO02/41802 and WO 02/11641 disclose toothbrushes with a liquid dentifrice container. US 5,909,977 discloses a dentifrice dispensing toothbrush utilizing a refillable cartridge for storing dentifrice material and a compressible elastic button for pumping dentifrice material to the brush head. Further development of this idea includes the use of hollow bristles through which the dentifrice flows as disclosed in US 5,309,590.

However, integration of this type creates new problems with regards to the Theological profile of the integrated oral composition. The oral composition is preferably pumped from a storage reservoir to the place of application through some form of tubing. In a toothbrush, the toothbrush neck needs to be less than a certain maximum diameter and greater than a certain minimum length to allow it to be comfortably used in the mouth. This restriction on the size of the neck necessarily restricts the maximum cross-sectional area and minimum length of the tubing used within to deliver the composition to the head of the toothbrush from the reservoir. This limitation on the cross-sectional area of the tubing results in high shear rates being developed as the oral composition is pumped through the tube, increasing the pressure required to transport the oral composition. Furthermore, toothbrushes necessarily have a limited pumping pressure, either due to power requirements in electrically operated pumping systems, or due to the maximum amount of force that can be applied conveniently by a consumer on manually operated pumps. Thus, the composition must have a specific rheological profile to allow it to be pumped through given tubing whilst minimising the pressure requirement of the pumping system.

Furthermore, available oral compositions are not optimised for use with oral treatment systems, and may lead to too little or excessive toothbrush head pressure being applied to the oral tissues by the consumer to clean the teeth. Application of too little or excessive pressure to the bristle head may result in the cleaning efficiency of the oral treatment system being reduced.

Therefore, it is desirable to provide compositions for use in oral treatment systems that can be easily pumped through tubes of given diameter and length. Furthermore, it is also desirable to provide an oral treatment system comprising dentifrice compositions that have rheological properties that enable the composition to be easily pumped, yet also allow it to be retained within the bristles when dispensed. Further still, it is desirable to provide a kit containing the apparatus required to use an oral composition in an oral treatment system.

Additionally, it is desirable to provide oral compositions optimised for use in oral treatment systems that enables the manipulation of and pressure exerted by the consumer on the oral treatment system when used in the mouth.

These and other objects of the present invention will become more readily apparent from consideration of the following summary, detailed description and examples.

**Summary of the Invention**

In the following description:

"integrated device" or "integrated oral treatment system" refers to a device or oral treatment system which comprises an in-built reservoir capable of containing an oral composition, such as a dentifrice, for delivery of the composition to the oral cavity.

"tube" means an enclosed internal cavity through which flowable materials may pass from one end to another;

"longitudinal axis" is the axis that is parallel to the direction of flow within the tube;

"internal cross-section" is the cross-section of the tube taken in the plane perpendicular to the longitudinal axis and enclosed by the internal face of the tubing material;

"length" refers to the length of the tube along the longitudinal axis; and

"pump return force" means the force exerted by or on the pump during the refilling portion of the pumping cycle. The pump return force can be determined by measuring the minimum force required to prevent the pump refilling following one expulsion cycle, and holding it at equilibrium.

The invention provides an oral composition comprising:

a) greater than 0.5% up to 2% thickening ingredients selected from xanthan gum, carrageenan and derivatives, gellan gum, hudroxypropyl methyl cellulose, sclerotium gum and derivatives, pullulan, rhamsan gum, welan gum, konjac, curdlan, and mixtures thereof;
b) from 1% to 10% by weight abrasive wherein the abrasive is a silica abrasive having an oil absorption of from 30 to 100 g per 100 g silica;
c) 30% to 60% total water; and
d) 30% to 60% humectants selected from polyols and polyethylene glycols;

the composition having a viscosity of less than 5 Pa.s at a shear rate of 450 $s^{-1}$; a viscosity of from 0.001 Pa.s to less than 780 Pa.s at a shear rate of 20 $s^{-1}$ and a post-shear viscosity of at least 10 Pa.s at a shear rate of 1 $s^{-1}$.
The oral compositions described herein have Radioactive Dentin Abrasion ("RDA") values of from 70 to 140.
All parts, percentages and proportions referred to herein and in the appended claims are by weight of the total oral composition unless otherwise indicated. All measurements are made at 25˚C on the total oral composition unless otherwise indicated. Viscosity as used herein is measured using a Cammed CSL2 100 rheometer with a 2 cm diameter parallel plate measuring system and a 500 micron gap between the plates. If, however, the composition contains a particulate ingredient which is insoluble in the composition and has a particle size of greater than 50 microns then a gap between the plates of ten times the particle size should be used. For the purpose of gap setting, an ingredient particle size should be taken as the smallest sieve mesh size through which at least 90% by weight of the dry ingredient passes.

**Detailed Description**

The oral compositions of the present invention are suitable for use in oral treatment systems wherein the oral composition is contained within a reservoir and is pumped from the reservoir to an outlet via a tube. They have viscosities that are optimised for transport via tubes of given diameter and length via relatively low force pumping means. Relatively low force means that the pumping force required is not sufficiently large to require high power input with regards to an electrically driven pump, or require exertion of high force from the finger of the user on the actuator of the pump. Relatively low force to transport the oral compositions refers to a force that is less than 100 N being required to drive the pump to attain the delivery rates indicated.
The oral composition for use herein enables the delivery of the oral composition through defined tube cross-sectional areas and lengths at a flow rate of at leash 0.1 ml/s, preferably 0.2 ml/s. This is advantageous in allowing quick and efficient transport of the oral composition from the reservoir to the applicator head. The oral compositions for use herein may be Newtonian compositions, or shear thinning compositions, preferably shear thinning such that, as the shear rate, and therefore shear stress, is increased the viscosity of the oral composition progressively lowers. The use of the oral composition in the oral treatment system herein allows efficient delivery of the oral composition to the applicator and its retention within the applicator, without requiring multiple applications or negatively influencing the aesthetics of the product.

*ORAL TREATMENT SYSTEM*

Toothbrush

The oral compositions of the present invention are suitable for use in a wide variety of oral treatment systems such as integrated, toothbrushes and electric toothbrushes. An exemplary oral treatment system is disclosed in WO 02/064056 A1 and US 6,402,410 B1. Such systems comprise a reservoir, an applicator and means for transporting the oral composition from the reservoir to the applicator. Preferably, the oral treatment system is a hand-held portable device suitable for use with one hand. The oral treatment system preferably comprises a unitary housing that can be comfortably gripped in the user's hand, the housing preferably comprising the reservoir and the means for transporting the oral composition.

Reservoir

Suitable reservoirs for storing the present oral compositions may be fixedly or removably attached to the treatment system. Preferably the reservoir is replaceably removable from the housing, for example to allow the reservoir to be refilled and the reservoir reinserted, or for the reservoir to be replaced by one of substantially similar construction that may comprise further quantities of oral composition. Any suitable reservoir may be utilised in the present invention. It should be understood that the reservoir utilized may be fully or partially internal to the housing of the treatment system, or fully or partially internal to the housing.

Non-limiting examples of suitable reservoirs include positive displacement type reservoirs that are generally rigid-walled such as a cartridge, and also include pump-evacuated type reservoirs that are generally soft-walled such as sachets, bladders, and blisters. Preferably, the reservoir is of the goft-walled, pump-evacuated type, more preferably a sachet. The reservoir may be manufactured from materials suitable to the application, known to those skilled in the art. For example, positive displacement type reservoirs may be made from metal, rigid plastic and other suitably hard materials. Soft-walled pump evacuated reservoirs are preferably manufactured out of soft, pliable plastics such as PET, PE, metallised PE, laminated aluminium and other suitable materials known to those skilled in the art. Preferably the reservoir defines an internal volume of from 5 ml to 25 ml.

In an embodiment, the reservoir comprises means for attachment to the pump. This is desirable to allow the reservoir to be attached to the pump, for example when a replacement reservoir is being attached to the oral treatment system. In another embodiment, the reservoir may comprise the pumping means and means for attachment to the transporting tubs. This is advantageous to allow the reservoir and pump to be replaced at the same time as a single unit. Suitable examples of the means for attachment of the reservoir to the pump include snap-lock fittings, gasket seals, bayonet and screw fittings.

Provision may be made for in situ refilling of the reservoir from a separate refill cartridge, external to the housing of the system, which is provided either with the system, or as a separate item. This can be advantageous where the volumes of composition consumed during treatment are relatively large and it would be costly or wasteful to throw away an internal reservoir each time it was emptied. The external refill cartridge may form part of a base station that additionally performs the function of providing a holder for the housing of the treatment system. Where the treatment system comprises electric means, the base station may additionally comprise recharging means for recharging a rechargeable battery within the treatment system.

Pump and Tubing

The oral treatment system comprises means for transporting the oral composition from the reservoir to the applicator. Means for transporting the oral compositions may comprise a pump and/or tubing. Preferably, the oral treatment system comprises a pump to provide mechanical pressure to transport the oral compositions from the reservoir to the applicator. The pump may comprise an electronically controlled pump or, for example, a resilient push button operated mechanical pump placed over the reservoir, or in line with a tube leading from the pump to the reservoir. Preferably the pump comprises a positive displacement pump. Positive displacement pumps (see for example p155 Chemical Engineering, Volume 6, Design, R.K. Sinnott, Permagon Press, 1983) cover a variety of different designs of pumps in which material cannot back-flow through the pump when it is not being activated. Specific positive displacement pump designs suitable for use in a device such as this include peristaltic pumps, pistons and diaphragm pumps.

Diaphragm pumps herein typically comprise a pump body in which is contained the pump chamber (a void which may be filled with the product to be pumped), a pump activator consisting of an elastomeric membrane which forms the surface of the pump chamber, an inlet valve and an outlet valve. The pump activator may additionally comprise a spring to aid its return to the maximum pump chamber volume following depression. The pump body also includes an inlet nozzle with channel connected to inlet valve and an outlet nozzle with channel connecting to the outlet valve. The inlet valve is positioned between the inlet nozzle and the pump chamber and opens when the pressure inside the pump is lower than that on the inlet side of the valve and closes when the pressure in the pump is higher than that on the inlet side of the valve. The inlet valve typically consists of an elastic material with the ability to spring shut on removal of reduced pressure in the chamber; a suitable material is PET. The outlet valve is positioned between the pump chamber and the outlet nozzle, and opens when the pressure inside the pump is higher than that on the outlet side of the valve and closes when the pressure in the pump is lower than that on the outlet side of the valve. The outlet valve typically has similar properties to the inlet valve. The valves may be mounted onto a valve housing to support their structure and ensure that they can only open in one direction.

The activator, when depressed, causes the volume of the pump housing to decrease, pressure in the pump housing to increase and product to be expelled through the outlet valve. The activator is sprung to allow it to recover to maximum volume when released. When the activator is released, the volume of the pump housing increases, causing the pressure in the housing to decrease and product to be sucked into the pump housing through the inlet valve. The activator typically consists of a thermoplastic elastomer e.g. TPE (polyetherester).

In another embodiment, the pump may comprise a piston. A piston such as a syringe negates the need for a pump to be refilled with product. Preferred herein are diaphragm pumps.

The oral treatment system herein comprises means to transport the oral composition from the reservoir via the pump to the applicator. Suitable transport means include tubes. Non-limiting examples of tubes include silicone tubing, moulded plastic channels, and plastic tubing. Preferably the tube has a sufficiently small internal cross-sectional area to allow it to be contained within the neck of the applicator without increasing the diameter of the neck of the applicator itself. The internal cross-section of the internal surface of the tube perpendicular to the longitudinal axis can define any closed shape, such as for example, a circle, an oval, or a polygon such as a square or rectangle.

In one embodiment, the tube has a sufficiently small internal cross-sectional area so as to be contained within the neck of the applicator along with a drive shaft for the motorised manipulation of the applicator head, the neck of the applicator being sufficiently narrow so as to maintain the ergonomics of the applicator. The internal cross-sectional area of the tube is preferably from 0.0025 $mm^2$ to 25 $mm^2$. More preferably, the internal cross-sectional area of the tube is from 0.01 $mm^2$ to 20.25 $mm^2$, more preferably still from 0.25 $mm^2$ to 16 $mm^2$. Yet more preferably still, the internal cross-sectional area of the tube is from 0.5 $mm^2$ to 10 $mm^2$.

Furthermore, the tube requires a length such that the reservoir and pump can be positioned at a distance from the applicator head so that the pump can be easily activated by the hand holding the oral treatment system. Preferably the tubing has a length of from 10 mm to 300 mm, more preferably from 50 mm to 250 mm, more preferably still from 100 mm to 200 mm. Yet more preferably the tube length is from 120 mm to 180 mm.

Additionally, the tube for use herein has a length to internal cross-sectional area (y:A) ratio of at least 1:1 $mm^{-1}$, preferably, at least 2:1 $mm^{-1}$, Where the tube has multiple different internal cross-sectional areas, the internal cross-sectional area used to determine the minimum length allowable by the above ratio shall be the smallest internal cross-sectional area of the tube. Where the tube expands under the flow of the oral composition, the internal cross-sectional area used to determine the minimum length according to the above ratio is that area defined by the maximum expansion of the tube.

Applicator

The oral treatment system includes an applicator for applying the oral composition to the oral cavity. The applicator may be fixedly or releasably attached to the housing of the oral treatment system. Preferably the applicator is releasably attached to the applicator. This is advantageous to allow replacement of the applicator with one of substantially similar construction without requiring the entire oral treatment system to be replaced. The applicator may be any device suitable for applying the oral compositions herein to the teeth or oral soft tissues, and may comprise a neck portion extending from the attachment means and terminating in a head portion that comprises an application surface that may be a brush or a sponge. Preferably the applicator head comprises a brush. Typically the neck portion has a length of from 20 mm to 100 mm and a diameter from 5 mm to 115 mm.

In addition to the tube, the applicator may comprise means for driving a motorised applicator head. Examples of such means include a drive shaft, or gear arrangement.

*ORAL COMPOSITION*

Viscosity

The oral composition of the present invention is a fluid composition having a select viscosity profile. Without wishing to be bound by theory, it is believed that the pressure required to pump an oral composition through a tube of given internal cross-sectional area and length is proportional to the viscosity of the oral composition at a shear rate defined by the tube size. Due to the limitations of pumping pressure, either to enable energy minimisation in the case of electrically powered pumps, or to prevent excessive force requirement for manually operated pumps, the oral compositions of the present invention preferably have a viscosity at 25°C not greater than $\eta$ (in Pa.s) at shear rate $\dot{\gamma}$ (in s-1), wherein $\eta$ and $\dot{\gamma}$ are defined by the equations:

$$\text{Equation 1:} \qquad \eta = \frac{250A^2}{4y} \quad \text{and} \quad \dot{\gamma} = \frac{2500}{A^{3/2}} \, ;$$

and wherein A and y are respectively the internal cross-sectional area ($mm^2$) and length (mm) of the tube used to transport the oral composition from the reservoir to the applicator head via a pump. Oral compositions having a viscosity of less than η at a shear rate $\dot{\gamma}$ will be suitable for pumping through a tube defined in terms of A and y above. Where the tube has multiple different internal cross-sectional areas, the viscosity shall be calculated as though the entire length of the tube (y) has an internal cross-sectional area (A) equal to the smallest internal cross-sectional area of the tube. Where the tube expands when the product contained within is pumped along its length, the cross-sectional area shall be determined as the area at the maximum expansion of the tube.

Table 1 below presents examples of preferred tube length and internal cross-sectional area combinations for use in the oral treatment system of the present invention, and the required viscosity limits for an oral composition for use therein according to equation 1.

**Table 1**

| Length (mm) | 50 | 100 | 120 | 150 | Shear Rate $\dot{\gamma}$ (s$^{-1}$) |
|---|---|---|---|---|---|
| Area (mm$^2$) | Maximum Viscosity (Pa.s) | | | | |
| 1 | 1.25 | 0.63 | 0.52 | 0.42 | 2500 |
| 4 | 20 | 10 | 8.33 | 6.67 | 312.5 |
| 9 | 101.25 | 50.63 | 42.19 | 33.75 | 92.59 |
| 16 | 320 | 160 | 133.33 | 106.67 | 39.06 |

Additionally, the oral compositions of the present invention have a viscosity at a shear rate of 20 s$^{-1}$ of from 0.001 Pa.s to 780 Pa.s, preferably from 0.1 Pa.s to 500 Pa.s more preferably from 1 Pa.s to 100 Pa.s.

Unless otherwise specified herein, viscosities as referred to herein are measured on the compositions of the present invention without any specific prior shear being applied to the composition, although it should be recognised that, in practice, a small amount of shear will inevitably have been applied to the composition as the Carrimed rheometer builds up to a particular programmed shear rate at which the measurement is taken. In order to simulate the effects of a more rigorous application of shear, such as that obtained when the composition is pumped through a tube to a brush head, the viscosity can also be measured on the Carrimed arter a defined programmed shear. The oral compositions of the present invention have a "post-shear" viscosity of at least 10 Pas at a shear rate of 1s$^{-1}$. As used herein, "post-shear viscosity" means the viscosity of the composition at 1 s$^{-1}$ following exposure of the composition to a linear sweep of shear rate from 0 to 450 s$^{-1}$ over a period of 30 seconds, followed by a second linear sweep of shear rate from 450 s$^{-1}$ to 0 s$^{-1}$ over a period of 30 seconds, the post-shear viscosity being determined as the viscosity at 1 s$^{-1}$ on the second shear rate sweep from 450 s$^{-1}$ to 0 s$^{-1}$. The samples are loaded onto the base plate of the Carrimed CSL2 100 rheometer using a spatula, following which the plates are brought together to the appropriate gap distance (usually 500 microns) using the exponentially decaying compression setting. The sample is then allowed to equilibrate for 5 minutes, following which the test protocol is initiated.

A post-shear viscosity of at least 10 Pa.s at a shear rate of 1 s$^{-1}$ prevents the oral composition from running off the applicator head once dispensed.

Preferably, the oral composition for use herein has a post-shear viscosity at 25°C of at least 25 Pa.s at a shear rate of 1 s$^{-1}$.

Furthermore, it is preferable that the oral compositions of the present invention have limited hysteresis, such that once the viscosity of the composition has been lowered by an increase in shear stress such as pumping through a tube, the composition returns rapidly to its low shear viscosity rapidly once the high shear stress has been removed. Preferably, the oral compositions of the present invention have a ratio of the initial viscosity, measured at a shear rate of 1 s$^{-1}$ during the initial shear rate sweep from 0 s$^{-1}$ to 450 s$^{-1}$, to the post-shear viscosity, measured at a shear rate of 1 s$^{-1}$ during the second shear rate sweep from 450 s$^{-1}$ to 0 s$^{-1}$ of less than 10:1, more preferably less than 5:1, more preferably still less than 2:1. It has been found that the ratio of initial viscosity to post-shear viscosity is an indicator of the level of hysteresis present in the oral compositions of the present invention. A ratio of greater than 10 indicates that the oral composition exhibits too much hysteresis following exposure to high shear, and therefore having limited return to its pre- high shear rate viscosity following removal of the shear stress.

Where the means for pumping the composition comprise a direct displacement pump, the oral composition preferably has a viscosity not greater than η (in Pa.s) at shear rate $\dot{\gamma}$ (in s$^{-1}$), wherein η and $\dot{\gamma}$ are defined by the equations:

$$\text{Equation 2:} \qquad \eta = \frac{10A^2}{y} \quad \text{and} \quad \dot{\gamma} = \frac{10,000}{A^{3/2}}.$$

Table 2 below presents examples of preferred tube length and internal cross-sectional area combinations for use in the oral treatment system of the present invention, and the preferred viscosity limits for an oral composition for use with a direct displacement pump therein according to equation 2.

Table 2

| Length (mm) | 50 | 100 | 120 | 150 | Shear Rate $\dot{\gamma}$ (s$^{-1}$) |
|---|---|---|---|---|---|
| Area (mm$^2$) | Maximum Viscosity (Pa.s) | | | | |
| 1 | 0.2 | 0.1 | 0.08 | 0.07 | 10000 |
| 4 | 3.2 | 1.6 | 1.33 | 1.07 | 1250 |
| 9 | 16.2 | 8.1 | 6.75 | 5.4 | 370.37 |
| 16 | 51.2 | 25.6 | 21.33 | 17.07 | 156.25 |

More preferably, where the means for pumping the composition comprises a positive displacement pump, the oral composition preferably has a viscosity not greater than $\eta$ (in Pa.s) at shear rate $\dot{\gamma}$ (in s$^{-1}$), wherein $\eta$ and $\dot{\gamma}$ are defined by the equations:

$$\text{Equation 3:} \qquad \eta = \frac{6A^2}{y} \quad \text{and} \quad \dot{\gamma} = \frac{10,000}{A^{3/2}}.$$

Table 3 below presents examples of preferred tube length and internal cross-sectional area combinations for use in the oral treatment system of the present invention, and the preferred viscosity limits for an oral composition for use with a positive displacement pump therein according to equation 3.

Table 3

| Length (mm) | 50 | 100 | 120 | 150 | Shear Rate $\dot{\gamma}$ (s$^{-1}$) |
|---|---|---|---|---|---|
| Area (mm$^2$) | Maximum Viscosity (Pa.s) | | | | |
| 1 | 0.12 | 0.06 | 0.05 | 0.04 | 10000 |
| 4 | 1.92 | 0.96 | 0.8 | 0.64 | 1250 |
| 9 | 9.72 | 4.86 | 4.05 | 3.24 | 370.37 |
| 16 | 30.72 | 15.36 | 12.8 | 10.24 | 156.25 |

More preferably, where the means for pumping the composition comprises a diaphragm pump, the oral composition preferably has a viscosity at not greater than $\eta$ (in Pa.s) at shear rate $\dot{\gamma}$ (in s$^{-1}$), wherein $\eta$ and $\dot{\gamma}$ are defined by the equations:

$$\text{Equation 4:} \qquad \eta = \frac{3A^2}{y} \quad \text{and} \quad \dot{\gamma} = \frac{10,000}{A^{3/2}}.$$

Table 4 below presents examples of preferred tube length and internal cross-sectional area combinations for use in the oral treatment system of the present invention, and the more preferred viscosity limits for an oral composition for use therein according to equation 4.

Table 4

| Length (mm) | 50 | 100 | 120 | 150 | Shear Rate $\dot{\gamma}$ (s$^{-1}$) |
|---|---|---|---|---|---|
| Area (mm$^2$) | Maximum Viscosity (Pa.s) | | | | |
| 1 | 0.06 | 0.03 | 0.025 | 0.02 | 10000 |
| 4 | 0.96 | 0.48 | 0.4 | 0.32 | 1250 |
| 9 | 4.86 | 2.43 | 2.03 | 1.62 | 370.37 |
| 16 | 15.36 | 7.68 | 6.4 | 5.12 | 156.25 |

More preferably still, where the means for pumping the composition comprises a diaphragm pump, the oral composition preferably has a viscosity at not greater than $\eta$ (in Pa.s) at shear rate $\dot{\gamma}$ (in s$^{-1}$), wherein $\eta$ and $\dot{\gamma}$ are defined by the equations:

$$\eta = \frac{10A^2}{4y} \quad \text{and} \quad \dot{\gamma} = \frac{10000}{A^{3/2}}.$$

In a more preferred embodiment, where the means for pumping the composition comprises a diaphragm pump, the oral composition preferably has a viscosity not greater than $\eta$ (in Pa.s) at shear rate $\dot{\gamma}$ (in s$^{-1}$), wherein $\eta$ and $\dot{\gamma}$ are defined by the equations:

$$\eta = \frac{3A^2}{4y} \quad \text{and} \quad \dot{\gamma} = \frac{10000}{A^{3/2}}.$$

Oral compositions having viscosities within the limits defined by the above equations are excellent for use in oral treatment systems comprising means for pumping the oral composition, wherein the oral composition is pumped from a reservoir to the applicator via a tube. Compositions with viscosities above those defined in the present application are too viscous to be efficiently pumped through a tube of defined diameter and length. Whilst these compositions may flow through a tube of the defined internal cross-sectional area and length, the force required to effectively transport these compositions with an efficient delivery flow rate is above that which can be efficiently supplied by a battery operated pump, or from manual manipulation of a pump activator by the consumer.

It is understood that instruments used to measure shear stress and shear rate (such as the Carrimed CSL2 100 rheometer used herein) are unable to attain some of the shear rates indicated by the equations for $\dot{\gamma}$. In these instances, the viscosity of the oral composition at those shear rates is determined using the Herschel-Bulkley model. The shear stress of the oral composition is determined as a function of shear rate over a range of shear rates from 0 to 450 s$^{-1}$, or the maximum shear rate that can be applied to the oral composition without it becoming partially fractured, using a minimum of 40 evenly distributed shear rates within the range. The data is modelled by fitting the equation:

$$\tau = \tau_0 + \kappa\dot{\gamma}^n,$$

wherein $\tau$ is the shear stress, $\tau_0$ is the yield stress, $\dot{\gamma}$ is the shear rate, $\kappa$ is the consistency (the viscosity at 1 s$^{-1}$), and n is shear index. Once the values of $\tau_0$, $\kappa$ and n have been determined, the following equation is applied at the shear rate required to predict the viscosity of the oral composition at the high shear rate:

$$\eta = (\tau_0 \times \dot{\gamma}^{-1}) + (\kappa\dot{\gamma}^{n-1}).$$

The oral compositions of the present invention have a viscosity of less than 5 Pa.s, more preferably still less than 2 Pa.s at a shear rate of 450 s$^{-1}$. It has surprisingly been found that the compositions for use herein require these low viscosities at high shear rates to ensure that they are able to be adequately dosed onto the bristle head of the oral care system. Compositions with high shear viscosities substantially higher than those herein have been found to have inadequate flow rates for dispensing.

Additionally, it is preferable that the oral composition for use in the present invention has a viscosity such that, once the pump, by its activation, has been purged of the oral composition contained therein, the oral composition has a flow rate into the pump of at least 0.2 ml/s, wherein the pump return force is less than 100 N. This is desirable to allow the pump to refill efficiently following dispensing of the product.

Additionally, it is preferable that the oral composition of the present invention, when diluted to a 16.67% slurry in water has a viscosity of greater than 0.1 Pa.s at a shear rate of 1 s$^{-1}$. This is advantageous to provide an oral composition which, when diluted in the oral cavity, has a viscosity that is high enough to enable its retention in the oral cavity without excessive manipulation or pressure application of the applicator by the consumer. More preferably, the oral composition for use according to the present invention has a viscosity when diluted to a 16.67% slurry in water of greater than 0.2 Pa.s at a shear rate of 1 s$^{-1}$, more preferably still greater than 0.3 Pa.s at a shear rate of 1 s$^{-1}$. It has been found that oral compositions with dilution viscosities greater than 0.1 Pa.s at a shear rate of 1 s$^{-1}$ induce greater consumer compliance and better cleaning efficiency. It is believed that this is a result of less pressure being exerted by the oral treatment system on the oral tissues by the consumer to retain the diluted composition in the mouth. Reduction of the exerted pressure results in a better and more efficient cleaning action, and decreased irritation of the oral tissues. These two features result in improved consumer experience and therefore increase consumer compliance.

The rheology of the oral composition is affected by the types and levels of thickeners, and also the type and levels of optional ingredients. It is known by those skilled in the art that different thickeners may be used at different levels to provide similar viscosity at one shear rate. However, the thickeners may display differing viscosity profiles over a range of shear rates. Some thickeners, or thickening systems, such as those comprising synthetic hectorite clay, display moderate to high levels of hysteresis, limiting the return of the viscosity of the composition to its initial viscosity at low shear rate following exposure to a high shear rate, yet are desirable as they are highly shear thinning. Other thickeners do not shear thin sufficiently to enable their use herein.

To maintain the shear thinning properties of the oral composition, it is preferable to avoid high levels of materials that excessively increase the high-shear viscosity. Examples of such materials include any Newtonian liquids such as glycerin, sorbitol syurp and hydrogenated starch hydrolysates (also known as hydrogenated glucose syrup). These materials, whilst being desirable as humectants and optional ingredients, when added at high levels can alter the thinning properties of the composition such as to render it unsuitable for use herein. Most liquid ingredients have a higher viscosity than the water they are usually replacing. This includes other humectants such as propylene glycol and polyethylene glycols (PEG). If replacing water with one of these ingredients, the oral composition will have a higher viscosity at both high and low shear rates.

Additionally, it is preferable to avoid high levels of particulate solids such as abrasives. These materials reduce the amount of shear thinning in the oral composition, making them unsuitable for transport via a pump and tube. It has been found that oral compositions comprising high levels of particulate solids are sometimes unable to be drawn into the pump from the reservoir, resulting in the pump becoming blocked.

Thickeners

The formulation needs to have sufficiently low viscosity at high shear rate to enable adequate flow rates through the integrated device, whilst having sufficiently high viscosity at low shear rates following exposure to a high shear rate in order to prevent the product flowing off the brush once dispensed. The optimal thickener or thickening system for an integrated device formulation should increase the fluid viscosity at low shear rate (1 s$^{-1}$) whilst adding as little as possible to the viscosity a higher shear rates. In order to meet these objectives, the thickener or thickening system should impart a high degree of pseudoplasticity to the formulation, causing the formulation to significantly decrease in viscosity as the shear rate is increased.

Typically, thickeners imparting the highest level of pseudoplasticity are those which form structure by charge-charge interactions or hydrogen-bonding such as the colloidal silicas and hectorite clays. From a flow rate standpoint, these materials have ideal characteristics, being highly shear thinning. Thickeners forming cross-linked networks, such as polysaccharide derivatives including xanthan gum or synthetic polymers including carbomer, also give a high degree of pseudoplasticity. Thickeners that build structure by chain entanglement alone, such as cellulose gum, are also pseudoplastic, but tend to have a lower level of pseudoplasticity than those having a three dimensional order.

Thickeners may be used singly, or in combination to form "thickening systems". Some thickeners e.g. hectorite allow phase separation of the compositions in which they are used in the absence of a second thickener. Similarly, there may be restrictions on the level at which an individual thickener can be employed, requiring the addition of a further thickeners to achieve the required rheology profile.

As described above, compositions comprising bentonite clay and hectorite clays such as laponite are highly shear thinning, and therefore desirable for use in the present invention. However, compositions comprising these thickeners display moderate to high levels of hysteresis, limiting their attainable post-shear viscosity after exposure to high shear rates. Furthermore, these thickeners are unsuitable for use with certain oral care actives, such as sodium fluoride. Compositions comprising the more preferred polysaccharide gums such as xanthan gum and carrageenan do not shear thin to the same extent as those comprising laponite or bentonite clay, but display less hysteresis following dispensing. It is therefore necessary to select appropriate thickeners and levels in order to achieve the rheology requirements established herein.

For a particular thickener or combination of thickeners, achieving the correct rheological profile to allow the product to have a suitable flow rate at high shear, and to maintain the structure of the composition after exposure to a high sheaf rate in order to remain in and/or on the bristles will be highly dependent upon the formulation level at which the thickener or combination of thickeners is employed. Typically, increasing the level of thickener will lead to an increase in both the low shear rate and high shear rate viscosities. Therefore, there is a window of thickener levels that allows the composition to be pumped and to be retained on the bustles. The optimal level or levels of thickener of a combination of thickeners will also be determined by the grade of material employed, typically as a function of molecular weight or polymer chain length, with longer chain lengths resulting in higher viscosity. The thickener may also exhibit synergistic interaction with other ingredients in the formulation such that the level required to attain the correct viscosities at both high and low shear rates is altered. Many other factors may govern the selection of a particular thickener in a particular formulation. A specific charge on the thickener may be required for example in order to avoid undesirable interactions with other ingredients.

Thickeners suitable for the present invention include organic thickeners. Organic thickeners include xanthan gum, carrageenan and derivatives, gellan gum, hydroxypropyl methyl cellulose, sclerotium gum and derivatives, pullulan, rhamsan gum, welan gum, konjac, curdlan, and mixtures thereof.

Amounts of thickeners range from greater than 0.5% up to 2% by weight.

Surfactants

The oral composition comprises preferably greater than about 0.1% by weight of a surfactant of mixture of surfactants. Surfactant levels cited herein are on a 100% active basis, even though common raw materials such as sodium lauryl sulphate may be supplied as aqueous solutions of lower activity.

The surfactant is important for oral cleaning, both through removal of dirt from surfaces and in foam generation to suspend removed dirt. Suitable surfactant levels are from about 0.1% to about 15%, preferably from about 0.25% to about 10%, more preferably from about 0.5% to about 5% by weight of the total composition. Suitable surfactants for use herein include anionic, amphoteric, non-ionic, zwitterionic and cationic surfactants, though anionic, amphoteric, non-ionic and zwitterionic surfactants (and mixtures thereof) are preferred.

Useful anionic surfactants herein include the water-soluble salts of alkyl sulphates and alkyl ether sulphates having from 10 to 18 carbon atoms in the alkyl radical and the water-soluble salts of sulphonated monoglycerides of fatty acids having from 10 to 18 carbon atoms. Sodium lauryl sulphate and sodium coconut monoglyceride sulphonates are examples of anionic surfactants of this type. Sodium lauryl sulphate is preferred. In preferred embodiments, the oral composition comprises at least about 0.125%, preferably at least about 0.5% anionic surfactant, more preferably at least about 2%.

Suitable cationic surfactants useful in the present invention can be broadly defined as derivatives of aliphatic quaternary ammonium compounds having one long alkyl chain containing from about 8 to 18 carbon atoms such as lauryl trimethylammonium chloride; cetyl pyridinium chloride; benzalkonium chloride; cetyl trimethylammonium bromide; diisobutylphenoxyethyl-dimethylbenzylammonium chloride; coconut alkyltrimethylammonium nitrite; cetyl pyridinium fluoride; etc. Certain cationic surfactants can also act as germicides in the compositions disclosed herein.

Suitable nonionic surfactants that can be used in the compositions of the present invention can be broadly defined as compounds produced by the condensation of alkylene oxide groups (hydrophilic in nature) with an organic hydrophobic compound which may be aliphatic and/or aromatic in nature. Examples of suitable nonionic surfactants include the poloxamers; sorbitan derivatives, such as sorbitan di-isostearate; ethylene oxide condensates of hydrogenated castor oil, such as PEG-30 hydrogenated castor oil; ethylene oxide condensates of aliphatic alcohols or alkyl phenols; products derived from the condensation of ethylene oxide with the reaction product of propylene oxide and ethylene diamine; long chain tertiary amine oxides; long chain tertiary phosphine oxides; long chain dialkyl sulphoxides and mixtures of such materials. These materials are useful for stabilising foams without contributing to excess viscosity build for the oral composition.

Zwitterionic surfactants can be broadly described as derivatives of aliphatic quaternary ammonium, phosphonium, and sulphonium compounds, in which the aliphatic radicals can be straight chain or branched, and wherein one of the aliphatic substituents contains from about 8 to 18 carbon atoms and one contains an anionic water-solubilising group, e.g., carboxy, sulphonate, sulphate, phosphate or phosphonate. Preferred zwitterionic surfactants include the betaine surfactants disclosed in US-A-5,180,577.

Liquid Carrier Materials

The oral composition of this invention comprises greater than about 50% liquid carrier materials. Water is present. Water employed in the preparation of commercially suitable oral composition should preferably be deionised and free of organic impurities. The compositions include from 30% to 60% water. These amounts of water include the free water which is added plus that which is introduced with other materials such as with sorbitol and with surfactant solutions.

The liquid carrier will further include one or more humectants, Suitable humectants include glycerin, sorbitol, and other edible polyhydric alcohols, such as low molecular weight polyethylene glycols at levels of from 30% to 60%. To provide the best balance of foaming properties and resistance to drying out, the ratio off total water to total humectant is preferably from about 0.65:1 to 1.5:1, preferably from about 0.85:1 to 1.3:1.

The high shear rate viscosities of the oral compositions herein are greatly affected by the viscosity of Newtonian liquids present in the composition. As used herein "Newtonian liquids" includes liquid materials conforming to the law that the homogeneous shearing stress is the product of the coefficient fo viscosity and rate of shear. These may be either purs liquids such as glycerin or water, or a solution of a solute in a solvent such as a sorbitol solution in water. The level of contribution of the Newtonian liquid to the viscosity of the non-Newtonian oral composition will depend upon the level at which the Newtonian liquid is incorporated. Water is typically present in a significant amount in an oral composition, and has a Newtonian viscosity of approximately 1 mPa.s at 25˚C. Humectants such as glycerin and sorbitol solutions typically have a significantly higher Newtonian viscosity than water. As a result, the total level of humectant, the ratio of water to humectant, and the choice of humectants, is critical to determining the high shear rate viscosity of the oral compositions.

Common humectants such as sorbitol, glycerin, polyethyleneglycols, propylene glycols and mixtures thereof may be used, but the specific levels and ratios used will differ depending on the choice of humectant. Sorbitol may be used, but due to its relatively high Newtonian viscosity, typically cannot be incorporated at levels above 45% by weight of the composition, as it contributes significantly to the high shear rate viscosity of the oral composition. Conversely, propylene glycol may be employed at higher levels as it has a lower Newtonian viscosity than sorbitol, and hence does not contribute as much to the high shear rate viscosity of the oral composition. Glycerin has an intermediate Newtonian viscosity in between that of sorbitol and polyethylene glycol.

Ethanol may also be present in the oral compositions. These amounts may range from 0.5 to 5%, optimally from 1.5 to 3.5% by weight. Ethanol can be a useful solvent and can also serve to enhance the impact of a flavour, though in this latter respect only low levels are usually employed. Noc-ethauolic solvents such as propylene glycol may also be employed. Also useful herein are low molecular weight polyethylene glycols.

Other components

The oral composition herein will typically comprise a variety of other components such as abrasives, fluoride ion sources, chelating agents, antimicrobials, thickeners, silicone oils and other adjuvants such as preservatives and colouring agents.

Abrasives

The oral composition of the present invention may comprise a dental abrasive. Abrasives serve to polish the teeth and /or remove surface deposits. The abrasive material contemplated for use herein can be any material which does not excessively abrade dentine.

The oral compositions described herein have Radioactive Dentin Abrasion ("RDA") values of at from 70 to 140, most preferably from about 80 to about 125. The RDA values are determined according to the method set forth by Hefferen, "Journal of Dental Research", July-August 1976, pp. 563-573. and described in the Wason US Patent Nos. 4,340,583, 4,420,312 and 4,421,527.

Non-abrasive materials, such as polyphosphates can also contribute to a RDA value. A RDA value can, however, be measured for an abrasive in the absence of these materials. In the compositions of the present invention it is preferred that the abrasives themselves have a RDA value of from about 70 to about 140, more preferably from about 80 to about 125 when used at a 5% loading. The RDA of an abrasive at a 5% loading can be measured by applying the following technique. A test composition is prepared comprising 50% glycerin, 44% water, 5% of the abrasive and 1% xanthan gum by mixing the xanthan gum into the glycerin, adding the water and mixing and then adding the abrasive and mixing. The RDA of this test composition can then be measured using the standard RDA method to provide a measurement of the RDA of the abrasive at a 5% loading.

An alternate measure of assessing the cleaning performance of the compositions herein is to assess their effect on accumulated pellicle. The oral compositions described herein preferably have also have Pellicle Cleaning Ratio ("PCR") values of at least about 70, preferably from about 70 to about 140, more preferably from about 80 to about 125. The PCR cleaning values are determined by the PCR test described in "In Vitro Removal of Stain With Dentifrice", G. K. Stookey, T. A. Burkhard and B. R, Schemerhorn. J. Dental Research, 61, 1236-9, 1982.

Silica dental abrasives of various types offer exceptional dental cleaning and polishing performance without unduly abrading tooth enamel or dentin. The silica abrasive can be precipitated silica or silica gels such as the silica xerogels described in Pader et al., US-A-3,538,230, issued March 2, 1970 and DiGiulio, US-A-3,862,307, June 21, 1975, for example silica xerogels marketed under the tradename "Syloid" by W. R. Grace & Company, Davison Chemical Division. Suitable precipitated silicas are include those marketed by INEOS under the trade names Sorbosil AC 43 and AC 33. The silicas have an oil absorption of from 30 g per 100 g to 100 g per 100g of silica. It has been found that silicas with low oil absorption levels are less structuring, and therefore do not build the viscosity of the oral composition to the same degree as those silicas that are more highly structuring, and therefore have higher oil absorption levels. As used herein, oil absorption is measured by measuring the maximum amount of linseed oil the silica can absorb at 25°C.

Suitable abrasive levels are from 1% to 10%. Abrasive levels from 3% to 5% are preferred. It has been found that oral compositions with high levels of abrasive do not have rheological properties suitable for use in the present invention. Without wishing to be bound by theory, it is believed that oral compositions comprising higher levels of abrasives, whilst having a good low-shear viscosity for stability once dispensed, do not thin sufficiently under higher shear rates to provide good pump refill and outflow characteristics.

### Fluoride ion sources

For anticaries protection, a source of fluoride ion will normally be present in the oral composition. Fluoride sources include sodium fluoride, potassium fluoride, calcium fluoride, stannous fluoride, stannous monofluorophosphate and sodium monofluorophosphate. Preferred is sodium fluoride. Suitable levels provide from 25 to 2500 ppm off available fluoride ion by weight of the liquid dentifrice.

### Chelating agents

Another preferred optional agent is a chelating agent, of value as an anticalculus agent. Suitable chelating agents include organic acids and their salts, such as tartaric acid and pharmaceutically-acceptable salts thereof, citric acid and alkali metal citrates and mixtures thereof. Chelating agents are able to complex calcium found in the cell walls of the bacteria, Chelating agents can also disrupt plaque by removing calcium from the calcium bridges which help hold this biomass intact. However, it is possible to use a chelating agent which has an affinity for calcium that is too high. This results in tooth demineralisation and is contrary to the objects and intentions of the present invention. Preferred chelating agents have a calcium binding constant of about $10^1$ to $10^5$ to provide improved cleaning with reduced plaque and calculus formation. The amounts of chelating agent suitable for use in the present invention are about 0.1% to about 2.5%, preferably from about 0.5% to about 2.5% and more preferably from about 1.0% to about 2.5%. The tartaric acid salt chelating agent can be used alone or in combination with other optional chelating agents.

Another group of agents particularly suitable for use as chelating agents in the present invention are the water soluble polyphosphates, polyphosphonates, and pyrophosphates which are useful as anticalculus agents. The pyrophosphate salts used in the present compositions can be any of the alkali metal pyrophosphate salts. An effective amount of pyrophosphate salt useful in the present composition is generally enough to provide at least 1.0% pyrophosphate ion, preferably from about 1.5% to about 6% of such ions. The pyrophosphate salts are described in more detail in Kirk & Othmer, Encyclopedia of Chemical Technology, Second Edition, Volume 15, Interscience Publishers (1968).

Preferred are the water soluble polyphosphates such as sodium tripolyphosphate, potassium tripolyphosphate and sodium hexametaphosphate. Other long chain anticalculus agents of this type are described in WO98/22079. Also preferred are the water soluble diphosphonates. Suitable soluble diphosphonates include ethane-1-hydroxy-1,1,-diphosphonate (EHDP) and aza-cycloheptane-diphosphonate (AHP). The tripolyphosphates and diphosphonates are particularly preferred as they provide both anti-tartar activity and stain removal activity without building viscosity as much as much as less water soluble chemical stain removal agents and are stable with respect to hydrolysis in water. Due to the limited abrasive load able to be included in the present compositions for reasons of maintaining low viscosity, the soluble polyphosphates and diphosphonates are beneficial as destaining actives. Without wishing to be bound by theory, it is believed that these ingredients remove stain by desorbing stained pellicle from the enamel surface of the tooth. Suitable levels of water soluble polyphosphates and diphosphonates are from about 0.1% to about 10%, preferably from about 1% to about 5% and more preferably from about 1.5% to about 3% by weight of the oral composition. More preferred for use herein are the alkali metal salts of tripolyphosphates.

Still another possible group of chelating agents suitable for use in the present invention are the anionic polymeric polycarboxylates. Such materials are well known in the art, being employed in the form of their free acids or partially or preferably fully neutralised water-soluble alkali metal (e.g. potassium and preferably sodium) or ammonium salts. Additional polymeric polycarboxylates are disclosed in US-A-4,138,477 to Gaffar and US-A-4,183,914 to Gaffar et al., and include copolymers of maleic anhydride with styrene, isobutylene or ethyl vinyl ether, polyacrylic, polyitaconic and polymaleic acids, and sulphoacrylic oligomers of MW as low as 1,000 available as Uniroyal ND-2.

Antimicrobials

Also useful for inclusion in the compositions of the present invention are antimicrobial agents. A wide variety of antimicrobial agents can be used, including stannous salts such as stannous pyrophosphate and stannous gluconate; zinc salt, such as zinc lactate and zinc citrate; copper salts, such as copper bisglycinate; quaternary ammonium salts, such as cetyl pyridinium chloride and tetradecylethyl pyridinium chloride; bis-biguanide salts; and nonionic antimicrobial agents such as triclosan. Certain flavour oils, such as thymol, may also have antimicrobial activity. Such agents are disclosed in U.S. Pat. No. 2,946,725, Jul. 26, 1960, to Norris et al. and U.S. Pat. No. 4,051,234, Sep. 27, 1977 to Gieske et al. Also useful is sodium chlorite, described in WO 99/43290.

Antimicrobial agents, if present, are typically included at levels of from about 0.01% to about 10%. It is preferred to keep the level of stannous and cationic antimicrobial agents to less than 5%, preferably less than 1% to avoid staining problems.

Preferred antimicrobial agents are non-cationic antimicrobial agent, such as those described in US 5,037,637. A particularly preferred antimicrobial agent is 2',4,4'-trichloro-2-hydroxy-diphenyl ether (triclosan).

Silicone oils

An optional ingredient in the present compositions is a silicone oil. Silicone oils can be useful as plaque barriers, as disclosed in WO 96/19191. Suitable classes of silicone oils include, but are not limited to, dimethicones, dimethiconols, dimethicone copolyols and aminoalkylsilicones, preferred silicone oils are selected from dimethicone copolyols and aminoalkylsilicones, more preferably from dimethicone copolyols. Silicone oils are generally present in a level of from about 0.1% to about 15%, preferably from about 0.5% to about 5%, more preferably from about 0.5% to about 3% by weight.

Other adjuvants

Sweetening agents such as sodium saccharin, sodium cyclamate, Acesulfame K, aspartame, sucrose and the like may be included at levels from about 0.1 to 5% by weight. Other additives may also be incorporated including flavours, preservatives, opacifiers and colorants. Typical colorants are D&C Yellow No. 10, FD&C Blue No. 1, FD&C Red No. 40, D&C Red No. 33 and combinations thereof. Levels of the colorant may range from 0.0001 to 0.1%.

**Examples**

The following examples will more fully illustrate embodiments of this invention.

| Oral Composition | | | | | | |
| --- | --- | --- | --- | --- | --- | --- |
| Example# | I | II | III | IV | V | VI |
| **Material Name** | Wt % | Wt % | Wt % | Wt % | Wt % | Wt % |
| Sorbitol (70%) | 41.9 | 56.0 | 41.9 | 52.0 | 39.0 | 43.0 |
| Glycerin | 10.0 | 10.0 | 10.0 | 10.0 | 10.0 | 10.0 |
| Sodium fluoride | 0.32 | 0.32 | 0.32 | 0.32 | 0.24 | - |
| Sodium monofluoro phosphate | - | - | - | - | - | 1.11 |
| Xanthan gum | 1,0 | 0.6 | 1.0 | 1.0 | 1.2 | 0.5 |
| Synthetic hectorite[1] | - | - | - | - | - | 0.6 |
| Cellulose gum[2] | - | - | - | - | - | - |
| Sodium alkyl sulphate, 28% | 9.0 | 9.0 | 9.0 | 9.0 | 9.0 | 9.0 |
| Precipitated silica | 3.0 | 10.0 | 10.0 | 5.0 | 3.0 | 1.0 |
| PEG-6 | 5.0 | 4.5 | 5.0 | - | 5.0 | - |

(continued)

| Oral Composition Example# | I | II | III | IV | V | VI |
|---|---|---|---|---|---|---|
| Material Name | Wt % | Wt % | Wt % | Wt % | Wt % | Wt % |
| PEG-12 | - | - | - | 3.0 | - | - |
| PEG-20 M | - | - | 1.3 | 4.0 | - | - |
| Trilosan | 0.3 | 0.3 | - | - | - | - |
| Sodium saccharin | 0.45 | 0.45 | 0.45 | 0.45 | 0.3 | 0.3 |
| Sodium tripolyphosphate | - | - | - | - | 1.92 | - |
| Flavour | 1.7 | 1.7 | 1.9 | 1.7 | 1.7 | 1.7 |
| Preservative | 0.1 | 0.1 | 0.1 1 | 0.1 | 0.1 | 0.1 |
| CI 42090 FD&C Blue No.1 | 0.003 | 0.002 | 0.002 | 0.002 | 0.002 | 0.002 |
| Water | to 100% | to 100% | to 100% | to 100% | to 100% | to 100% |
| Viscosity at 1 s$^{-1}$ (Pa.s) | >10 | >10 | >10 | >10 | >10 | |
| Viscosity at 20 s$^{-1}$ (Pa.s) | <780 | <780 | <780 | <780 | <780 | |

1 Laponite D
2 Blanose 7M8SF

The oral compositions of the examples above were packaged in a vacuum-evacuated collapsible sachet made from heat sealed slummium-plastic laminate. The sachet was attached to the inlet nozzle of a diaphragm pump with a diaphragm chamber volume of 0.3 ml via a snap-lock connector. The outlet nozzle of the pump was connected to silicone tube with a circular cross-section, an internal cross-sectioaal area of 3.14 mm$^2$ and a length of 120 mm. The end of the silicone tube distal to the pump was finished with a silicone slit non-return valve in the head of a brush applicator. The entire sachet / pump / tube assembly was contained within the housing of an electric toothbrush sized for comfortable gripping by a user's hand, such that dome of the diaphragm pump was manually depressible from outside of the housing in order to pump the composition. When the plump dome was manually depressed the oral compositions of the examples above were dispensed at a flow rate of approximately 0.3 ml/s without need of excessive application force by the user. Once dispensed, the oral compositions rabidly attained an acceptable viscosity at 1 s$^{-1}$.

## Claims

1.  An oral composition for use in a reservoir of a hand-held toothbrush, wherein the composition is pumped through tubing from the reservoir to an outlet of the toothbrush, the oral composition comprising:

    a) from greater than 0.5% up to 2% by weight of thickening ingredients selected from xanthan gum, carrageenan and derivatives, gellan gum, hydroxypropyl methyl cellulose, sclerotium gum and derivatives, pullulan, rhamsan gum, weian gum, konjac, curdlan, and mixtures thereof;
    b) from 1% to 10% by weight abrasive wherein the abrasive is a silica abrasive having an oil absorption of from 30 to 100 per 100 g silica;
    c) from 30% to 60% by weight total water;
    d) frog 30% to 60% by weight humectants selected from polyols and polyethylene glycols;

    the composition having an RDA of fiom 70 to 140, a viscosity of less than 5 Pa.s at a shear rate of 450 s$^{-1}$, a viscosity of from 0.001 Pa.s to less than 780 Pa.s at a shear rate of 20 s$^{-1}$ and a post-shear viscosity of at least 10 Pa.s at a shear rate of 1 s$^{-1}$.

2.  The oral composition according to Claim 1 comprising from 1% to 5% by weight of an anti-tartar agent selected from alkali metal salts of water soluble tripolyphosphates and diphospbonates.

3.  The oral composition according to Claim 1 comprising from 3% to 5% by weight abrasive.

4.  A sachet, containing an oral composition according to any preceding claim.

**5.** A sachet according to Claim 4 defining an internal volume of from 5 to 25 ml.


**Patentansprüche**

**1.** Mundpflegezusammensetzung zum Gebrauch in einem Behälter einer handgehaltenen Zahnbürste, wobei die Zusammensetzung durch Röhrchen von dem Behälter zu einem Auslass der Zahnbürste gepumpt wird, wobei die Mundpflegezusammensetzung Folgendes umfasst:

a) von mehr als 0,5 Gew.-% bis zu 2 Gew.-% verdickende Inhaltsstoffe, ausgewählt aus Xanthangummi, Carrageenan und Derivaten, Gellangummi, Hydroxypropylmethylcellulose, Sclerotiumgummi und Derivate, Pullulan, Rhamsangummi, Welangummi, Konjac, Curdlan, und Mischungen davon;
b) von 1 Gew.-% bis 10 Gew.-% Schleifmittel, wobei das Schleifmittel ein Silica-Schleifinittel mit einer Ölabsorption von 30 bis 100 g pro 100 g Silica ist;
c) von 30 Gew.-% bis 60 Gew.-% Wasser gesamt;
d) von 30 Gew.-% bis 60 Gew.-% Feuchthaltemittel, ausgewählt aus Polyolen und Polyethylenglycolen;

wobei die Zusammensetzung einen RDA-Wert von 70 bis 140, eine Viskosität von weniger als 5 Pa.s bei einer Schergeschwindigkeit von 450 s$^{-1}$, eine Viskosität von 0,001 Pa.s bis weniger als 780 Pa.s bei einer Schergeschwindigkeit von 20 s$^{-1}$ und eine Nachscherviskosität von mindestens 1,0 Pa.s bei einer Schergeschwindigkeit von 1 s$^{-1}$ aufweist.

**2.** Mundpflegezusammensetzung nach Anspruch 1, umfassend von 1 Gew.-% bis 5 Gew.-% ein Antizahnsteinmittel, ausgewählt aus Alkalimetallsalzen von wasserlöslichen Tripolyphosphaten und Diphosphonaten.

**3.** Mundpflegezusammensetzung nach Anspruch 1, umfassend von 3 Gew.-% bis 5 Gew.-% Schleifmittel.

**4.** Beutel, der eine Mundpflegezusammensetzung nach einem der vorstehenden Ansprüche enthält.

**5.** Beutel nach Anspruch 4, der ein Innenvolumen von 5 bis 25 ml eingrenzt.


**Revendications**

**1.** Composition orale pour une utilisation dans un réservoir d'une brosse à dents manuelle, où la composition est pompée à travers un tubage du réservoir vers une sortie de la brosse à dents, la composition orale comprenant :

a) de plus de 0,5 % jusqu'à 2 % en poids d'ingrédients d'épaississement choisis parmi la gomme de xanthane, la carraghénine et dérivés, la gomme gellane, l'hydroxypropylméthylcellulose, la gomme de sclérotium et dérivés, la pullulane. la gomme de rhamsane, la gomme welan, le konjac, le curdlan et leurs mélanges ;
b) de 1 % à 10 % en poids d'abrasif, l'abrasif étant un abrasif à base de silice ayant une absorption d'huile allant de 30 à 100 g par 100 g de silice ;
c) de 30 % à 60 % en poids d'eau totale;
d) de 30 % à 60 % en poids d'humectants choisis parmi les polyols et polyéthylène glycols ;

la composition ayant une abrasion de la dentine (RDA) allant de 70 à 140, une viscosité de moins de 5 Pa.s à une vitesse de cisaillement de 450 s$^{-1}$, une viscosité allant de 0,001 Pa.s à moins de 780 Pa.s à une vitesse de cisaillement de 20 s$^{-1}$ et une viscosité après cisaillement d'au moins 10 Pa.s à une vitesse de cisaillement de 1 s$^{-1}$.

**2.** Composition orale selon la revendication 1, comprenant de 1 % à 5 % en poids d'un agent antitartre choisi parmi les sels de métal alcalin de tripolyphosphates et diphosphonates hydrosolubles.

**3.** Composition orale selon la revendication 1, comprenant de 3 % à 5 % en poids d'abrasif.

**4.** Sachet, contenant une composition orale selon l'une quelconque des revendications précédentes.

**5.** Sachet selon la revendication 4, définissant un volume interne allant de 5 à 25 mL.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3217720 A
- WO 0241801 A
- WO 0241802 A
- WO 0211641 A
- US 5909977 A
- US 5309590 A
- WO 02064056 A1
- US 6402410 B1
- US 5180577 A
- US 4340583 A, Wason
- US 4420312 A
- US 4421527 A
- US 3538230 A, Pader
- US 3862307 A, DiGiulio
- WO 9822079 A
- US 4138477 A, Gaffar
- US 4183914 A, Gaffar
- US 2946725 A, Norris
- US 4051234 A, Gieske
- WO 9943290 A
- US 5037637 A
- WO 9619191 A

### Non-patent literature cited in the description

- **R.K. Sinnott.** Chemical Engineering. Permagon Press, 1983, vol. 6
- **Hefferen.** *Journal of Dental Research,* July 1976, 563-573
- **G. K. Stookey ; T. A. Burkhard ; B. R, Schemerhorn.** In Vitro Removal of Stain With Dentifrice. *J. Dental Research,* 1982, vol. 61, 1236-9
- **Kirk ; Othmer.** Encyclopedia of Chemical Technology. Interscience Publishers, 1968, vol. 15